# EUROPÄISCHE PATENTSCHRIFT

(11) **EP 1 855 635 B1**
(45) Veröffentlichungstag und Bekanntmachung des Hinweises auf die Patenterteilung: **19.06.2013**
(21) Anmeldenummer: 05815231.5
(22) Anmeldetag: 06.12.2005
(51) Int. Cl.: A61K 8/44, A61K 8/365, A61K 8/25, A61K 8/34, A61K 8/49, A61K 8/97, A61Q 19/04, A61Q 17/04

(54) **KOSMETISCHES PRÄPARAT ZUR BRÄUNUNGSVERLÄNGERUNG**
COSMETIC PREPARATION FOR EXTENDING TANNING
PREPARATION COSMETIQUE POUR PROLONGER LE BRONZAGE

(30) Priorität: 17.12.2004 DE 102004062170
(43) Veröffentlichungstag der Anmeldung: 21.11.2007
(73) Patentinhaber: Coty Germany GmbH, 55116 Mainz (DE)
(72) Erfinder: GOLZ-BERNER, Karin, MC-98000 Monaco (MC); ZASTROW, Leonhard, MC-98000 Monaco (MC)
(74) Vertreter: Ziebig, Marlene
(86) Internationale Anmeldenummer: PCT/EP2005/013197
(87) Internationale Veröffentlichungsnummer: WO 2006/066741

(56) Entgegenhaltungen:
- US-A- 4 419 343
- US-A- 5 075 102
- ISP: "Sun screens and UV Protection," ISP : IN THE NEWS, [Online] 2004, Seiten 1-2, XP002366081 Gefunden im Internet: URL:www.ispcorp.com/innews/technical/cosms cience/04.html> [gefunden am 2006-02-03]

## Beschreibung

Die Erfindung betrifft ein kosmetisches Präparat zur Verlängerung der Hautbräunung, die durch natürliche Sonneneinstrahlung entstanden ist.

Phototan® LS 2261 E ist ein bekanntes Mittel zur Stimulierung der enzymatischen Aktivität von Dopa-Oxidase und beschleunigt die Bildung epidermischer Melaninpigmente. Es wird daher als Beschleuniger des Sonnenbräunungsprozesses eingesetzt sowie als Sonnenbräunungsverstärker.

Phototan® besteht aus einem Gemisch von Sorbitol, Arginin HCl, Ornithin HCl, Tyrosin und Silicagel. Dieses Gemisch soll nach Angaben des Herstellers in Konzentrationen von 3 bis 5 Gew-% eingesetzt werden und kann auch eine bestehende Sonnenbräunung verstärken, maximal jedoch um etwa 3-4 Tage.

Der Erfindung liegt die Aufgabe zugrunde, die vorhandene Sonnenbräunung für mehr als 3-4 Tage zu einem wesentlichen Prozentsatz erhalten und damit eine langanhaltende Wirksamkeit zu erreichen.

Erfindungsgemäß wird diese Aufgabe durch einen Sonnenbräunungsverlängerer gemäß Anspruch 1 gelöst, der als wirksamen Bestandteil einen Komplex aus
0,1 bis 3,0 Gew-% Caffein,
0,2 bis 2 Gew-% Phototan®,
0,01 bis 1,0 Gew-% Kupfergluconat
und weitere kosmetische Hilfsstoffe/Trägerstoffe, weitere Wirkstoffe und Gemische davon bis zu 100 Gew-%, jeweils bezogen auf das Gesamtgewicht des Präparates, enthält.

Vorteilhafte Ausführungsformen der Erfindung sind in den Unteransprüchen aufgeführt.

Es wurde gefunden, dass die Kombination der o.g. Substanzen in einer geeigneten Formulierung als Emulsion oder als Gel zu einer Sonnenbräunungsverlängerung und damit -verstärkung bis zu 7 bis 9 Tagen führt.

Die in dieser Kombination eingesetzten Substanzen wie Caffein und Kupfergluconat ließen eine derartig verstärkende Wirkung nicht erwarten.

Von Caffein ist bekannt, dass es eine gewisse durchblutungsfördernde Wirkung hat und in der Kosmetik zur Behandlung von Akne, Cellulitis und Haarausfall eingesetzt werden kann.

Kupfergluconat wird für die Zellregenerierung als entzündungswidriges Mittel, als Immunmodulator und als Aktivator für Lysyloxidase für die Kollagensynthese sowie als Cofaktor von Tyrosinase für die Melaninsynthese eingesetzt. Kupfergluconat hat gewisse bräunungsaktivierende Eigenschaften, die jedoch insgesamt für sich allein zu schwach sind, eine signifikante Bräunungsverstärkung zu bewirken.

Als weiterer Wirkstoff kann in dem erfindungsgemäßen Präparat die Aminosäure Glycin eingesetzt werden, die kosmetisch z.B. in Haut- und Haarkonditionierungsmitteln verwendet wird. Eine weitere Ausführungsform der Erfindung enthält zusätzlich einen Fruchtextrakt von Citrus aurantium. Beide Zusätze können die Sonnenbräunungsverstärkung auf bis zu 11 Tage verlängern und stellen damit eine bevorzugte Ausführungsform der Erfindung dar.

Besonders bevorzugte kosmetische Feuchthaltemittel für das erfindungsgemäße Präparat sind Propylenglycol, Glycerin und z.B. Lubragel® und Gemische davon.

Als Wirkstoffe mit radikalfangenden Eigenschaften können eingesetzt werden Pflanzenextrakte, Vitamine, Flavone, Flavonoide.

Besonders bevorzugt ist ein Gemisch aus Pflanzenextrakten, das als radikalfangende Komponente 0,4 bis 1,5 Gew-% eines Pflanzenextraktgemisches auf alkoholischer Basis enthält, bestehend aus den Extrakten von grünen Kaffeebohnen, Angelikawurzel, Camellia sinensis-Blättern und Pongamia pinnata (WO 2004/105706).

Ein weiterer Zusatz für das erfindungsgemäße kosmetische Präparat ist eine Wirkstoffzubereitung gemäß WO99/66881 mit hohem Radikalschutzfaktor mit einem Gehalt an einem durch Extraktion der Rinde von Quebracho blanco und nachfolgender enzymatischer Hydrolyse gewonnenem Produkt, das wenigstens 90 Gew-% Proanthocyanidin-Oligomere und höchstens 10 Gew-% Gallussäure enthält, in Mikrokapseln, sowie einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser.

Der Gehalt an Phototan® kann vorzugsweise im Bereich von 0,4 bis 0,8 Gew-% liegen.

Der Gehalt der radikalfangenden Komponente (Pflanzenextrakt aus grünen Kaffeebohnen, Angelikawurzel, Camellia sinensis-Blättern und Pongamia pinnata) kann vorzugsweise 0,4 bis 1,5 Gew-% betragen.

Besonders bevorzugt ist ein Caffeingehalt von 0,1 bis 2,0, insbesondere 0,1 bis 0,5 Gew-%.

Das erfindungsgemäße Präparat kann weiterhin kosmetische Hilfs- und Trägerstoffe enthalten, wie sie üblicherweise in solchen Zubereitungen verwendet werden, z.B. Wasser, Konservierungsmittel, Farbstoffe, Pigmente mit färbender Wirkung, Verdickungsmittel, Duftstoffe, Alkohole, Polyole, Ester, Elektrolyte, Gelbildner, polare und unpolare Öle, Polymere, Copolymere, Emulgatoren, Wachse, Stabilisatoren.

Zu den kosmetischen Wirkstoffen gehören z. B. anorganische und organische Lichtschutzmittel, Radikalfänger, Feuchthaltemittel, Vitamine, Enzyme, pflanzliche Wirkstoffe, Polymere, Antioxidationsmittel, entzündungswidrige natürliche Wirkstoffe und Erweichungsmittel.

Ein bevorzugter weiterer Wirkstoff ist beispielsweise ein mit sphärischem SiO₂ oder TiO₂ modifiziertem Kaolin gemäß WO96/17588 Beispiel 1 oder ein oder mehrere Antioxidationsmittel, Flavone, Flavonoide oder Gemische von Wirkstoffen dieser Gruppe.

Zu Antioxidationsmitteln und Radikalfängern gehören Vitamine wie Vitamin C und Derivate davon, beispielsweise Ascorbylacetate, -phosphate und -palmitate; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Carotinoide und Carotine, wie z.B α-Carotin, ß-Carotin; Harnsäure und Derivate davon; α-Hydroxysäuren wie Citronensäure, Milchsäure, Apfelsäure; Stilbene und deren Derivate.

Es ist weiterhin vorteilhaft, den erfindungsgemäßen Zusammensetzungen entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion, Butyl Methoxybenzoylmethane oder Menthyl Anthranilate.

Besonders bevorzugt sind Benzophenone-3, Butyl Methoxydibenzoylmethane, Octyl Methoxycinnamate, Octyl Salicylate, 4-Methylbenzylidene Camphor, Homosalate, Octocrylene, Ethylhexyl Methoxycinnamate, Isoamyl-p-Methoxycinnamate, Octyl Dimethyl PABA, Ethylhexyltriazone, Diethylhexyl Butamido Triazone, Ethylhexyl Salicylate, Methylene Bis-Benzotriazolyl Tetramethylbutylphenol, Disodium Phenyl Dibenzimidazole Tetrasulfonate, Bis-Ethylhexyloxyphenol Methoxyphenyl Triazine.

Weiterhin einsetzbar als Sonnenschutzfilter sind anorganische Pigmente auf Basis von Metalloxiden, wie TiO₂, SiO₂, ZnO, Fe₂O₃, ZrO₂, MnO, Al₂O₃, die auch im Gemisch verwendet werden können.

Kosmetische Zusammensetzungen mit der erfindungsgemäßen Wirkstoffzubereitung können als O/W- oder W/O-Emulsionen vorliegen. Geeignete Emulgatoren für O/W-Emulsionen sind beispielsweise Anlagerungsprodukte von 2-30 Mol Ethylenoxid an lineare C₈-C₂₂-Fettalkohole, an C₁₂-C₂₂-Fettsäuren und an C₈-C₁₅-Alkylphenole; C₁₂-C₂₂-Fettsäuremono- und -diester von Anlagerungsprodukten von 1-30 Mol Ethylenoxid an Glycerin.

Geeignete Emulgatoren für W/O-Emulsionen sind beispielsweise Anlagerungsprodukte von 2-15 Mol Ethylenoxid an Ricinusöl; Ester von C₁₂-C₂₂-Fettsäuren und Glycerin, Polyglycerin, Pentaerythrit, Zuckeralkohole (z.B. Sorbit), Polyglucoside (z.B. Cellulose); Polyalkylenglycole; Wollwachsalkohole; Copolymere von Polysiloxan-Polyalkylpolyether.

Die für die Erfindung eingesetzten Öle können übliche kosmetische Öle sein, wie ein Mineralöl; hydriertes Polyisobuten; synthetisches oder aus Naturprodukten hergestelltes Squalan; kosmetische Ester oder Ether, die verzweigt oder unverzweigt, gesättigt oder ungesättigt sein können; pflanzliche Öle; oder Gemische zweier oder mehrerer davon.

Besonders geeignete Öle sind beispielsweise Siliconöle, Mineralöle, Hydrogenated Polyisobuten, Polyisopren, Squalane, Tridecyltrimellitat, Trimethylpropantriisostearat, Isodecylcitrat, Neopentylglycoldiheptanoat, PPG-15-stearylether sowie pflanzliche Öle, wie Calendulaöl, Jojobaöl, Avocadoöl, Macadamianußöl, Rizinusöl, Kakaobutter, Kokosnußöl, Maisöl, Baumwollsamenöl, Olivenöl, Palmkernöl, Rapssamenöl, Safloröl, Sesamsamenöl, Sojabohnenöl, Sonnenblumensamenöl, Weizenkeimöl, Traubenkernöl, Kukuinußöl, Distelöl und Gemische davon.

Je nachdem.welche Öle ausgewählt werden, werden die kosmetischen Eigenschaften der festen Zusammensetzung beeinflußt, wie Transparenzgrad, Weichheit, Härte, Spreitungswirkung.

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Isopropylmyristat, Isopropylpalmitat, Oleylalkohol, Isopropyllaurat, Decyloleat, Octadecan-2-ol, Isocetylalkohol, Cetylpalmitat, Siliconöle wie Dimethylpolysiloxan, Polyethylenglycol, Lanolin, Kakaobutter, pflanzliche Öle wie Maisöl, Baumwollsamenöl, Olivenöl, mineralische Öle, Butylmyristat, Palmitinsäure usw.

Geeignete Feuchthaltemittel sind beispielsweise Glycerin, Butylenglycol, Propylenglycol und Gemische davon.

Als Farbpigmente, Pigmentgemische oder Pulver mit pigmentartiger Wirkung, worunter auch solche mit Perlglanz-Effekt zu verstehen sind, können zum Beispiel eingesetzt werden: Eisenoxide, natürliche Aluminiumsilicate wie Ocker, Titan(di)oxid, Glimmer, Kaolin, manganhaltige Tone wie Umbra und roter Bolus, Calciumcarbonat, Talkum, Glimmer-Titanoxid, Glimmer-Titanoxid-Eisenoxid, Glimmer-Titanoxid-organischer Farbstoff und Gemische davon.

Die Verwendung der erfindungsgemäßen kosmetischen Zusammensetzungen kann z.B. erfolgen in Form von Sonnencremes, Sonnengelen, After-sun-Produkten, Pre-sun-Produkten, Tagescremes, Nachtcremes, Körperlotionen, Reinigungsmilch, Körperpuder, Augenkosmetik, und in Produkten der dekorativen Kosmetik wie Deo-Stiften, Parfüm-Stiften, Lippenstiften, Gelen, Lidschatten, Kompaktprodukten wie Kompaktpuder oder Kompaktwachs, Rouge, Grundierung, Make-up usw. Die Herstellung derartiger Produkte erfolgt auf eine Weise, wie sie dem Fachmann auf diesem Gebiet bekannt ist.

Ein weiterer Gegenstand der Erfindung ist die Verwendung eines kosmetischen Präparates, enthaltend 0,1 bis 3,0 Gew-% Caffein, 0,2 bis 2 Gew-% Phototan®,0,01 bis 1,0 Gew-% Kupfergluconat und weitere kosmetische Hilfsstoffe/-Trägerstoffe, weitere Wirkstoffe und Gemische davon bis zu 100 Gew-%, jeweils bezogen auf das Gesamtgewicht des Präparates, zum Auftragen auf durch natürliche Sonneneinstrahlung gebräunte Haut, wobei das Auftragen wenigstens zweimal täglich mit einem Mindestabstand von 10 Stunden erfolgt.

Bevorzugt erfolgt die Verwendung zusammen mit einem weiteren Wirkstoff, wobei der Wirkstoff ausgewählt ist unter Glycin oder einem Extrakt von Citrus aurantium-Früchten.

Die Erfindung soll nachstehend durch Beispiele näher erläutert werden. Alle Angaben erfolgen in Gewichtsprozent, sofern nichts anderes angegeben ist.

### Beispiel 1 Bräunungsverstärker-Creme

| **Phase A** | |
|---|---|
| H₂O | q.s. ad 100 |
| Carbomer | 2,0 |
| Glycerin | 4,0 |

| **Phase B** | |
|---|---|
| Steareth 2 | 4,0 |
| Steareth 21 | 3,0 |
| Cetearylalkohol | 1,5 |
| Mineralöl | 1,5 |

| **Phase C** | |
|---|---|
| Triethanolamin | 2,0 |

| **Phase** D | |
|---|---|
| Phototan® | 0,8 |
| Kupfergluconat | 0,01 |
| Caffein | 0,5 |

| **Phase E** | |
|---|---|
| RPF-Komplex* | 1,0 |

| **Phase F** | |
|---|---|
| Parfümöl | 0,5 |
| Konservierungsmittel | 0,5 |

| | |
|---|---|
| * gemäß WO99/66881 Beispiel 2 | |

Die Phasen A und B werden separat bei etwa 75 °C hergestellt und dann unter Rühren miteinander vereinigt. Nach Abkühlen des Gemisches auf 50 °C wird die Phase C unter Rühren zugegeben. Nach weiterem Abkühlen auf etwa 35 °C werden die Phasen D und E hinzugesetzt. Schließlich wird bei etwa 30 °C die Phase hinzugesetzt und das Gemisch etwa 20 Minuten homogenisiert.

### Beispiel 2 Lotion

| **Phase A** | |
|---|---|
| H₂O | q.s. ad 100 |
| Glycerin | 4,0 |

| **Phase B** | |
|---|---|
| PEG-20 Methyl Glycose Sesquistearate | 2,0 |
| Tocopherol | 3,2 |
| Petrolatum | 2,0 |
| Squalane | 6,0 |
| Silicone | 2,0 |

| **Phase C** | |
|---|---|
| Phototan® | 0,5 |
| Kupfergluconat | 0,01 |
| Caffein | 0,1 |
| Glycin | 0,01 |

| **Phase D** | |
|---|---|
| RPF-Komplex* | 1,0 |

| **Phase E** | |
|---|---|
| Parfüm | 0,5 |
| Konservierungsmittel | 0,5 |

| | |
|---|---|
| * gemäß WO99/66881 Beispiel 1 | |

Die separat hergestellten Phasen A und B werden bei etwa 65 °C zusammengerührt. Anschließend werden mit max. 800 U/min die Phase C bei 35 °C und die Phasen D und E bei 30 °C zu dem Gemisch gegeben und zum Abschluss mehrere Minuten homogenisiert.

### Beispiel 3 Nachtcreme

| **Phase A** | |
|---|---|
| H₂O | q. s. ad 100 |
| Carbomer | 0,1 |

| **Phase B** | |
|---|---|
| Stearinsäure | 2,0 |
| Cetearylalkohol | 2,0 |
| Glyceryl Stearate | 2,0 |

| **Phase C** | |
|---|---|
| Triethanolamin | 0,1 |

| **Phase D** | |
|---|---|
| Phototan® | 0,4 |
| Kupfergluconat | 0,01 |
| Caffein | 2,0 |
| Glycin | 0,01 |
| Biotanning®¹ | 1,0 |

| **Phase E** | |
|---|---|
| RPF-Komplex* | 0,5 |

| **Phase F** | |
|---|---|
| Parfümöl | 0,5 |
| Konservierungsmittel | 0,5 |

| | |
|---|---|
| * gemäß WO99/66881 Beispiel 1 ¹ INCI: Hydrolyzed Citrus Aurantium Dulcis Fruit Extract Die Verfahrensweise entspricht der von Beispiel 1. | |

### Beispiel 4 Vergleichsversuch

Für eine Gruppe von 10 Probandinnen im Alter von 18 bis 43 Jahren mit Mischhaut wurde die Bräunungsverlängerung mittels eines erfindungsgemäßen Präparats gemessen. Die Probandinnen hatten eine etwa gleiche deutliche Hautbräunung durch natürliche Sonnenbräune. Der Ausgangswert und die Folgewerte wurden mittel MX18 Mexameter® (Courage + Khazaka electronic GmbH, Deutschland) auf dem Unterarm gemessen. Der Unterarm der Probandinnen war im Testzeitraum von 14 Tagen keiner UV-Strahlung ausgesetzt.

Von jedem Messpunkt wurden 8 Messwerte für den Melaninwert ermittelt. Von den Probandinnen wurden die kosmetischen Präparate wie folgt aufgebracht:
Probandin 1-5 Lotion von Beispiel 2 morgens und abends
Probandin 6-10 Creme von Beispiel 1 morgens und abends.

In der folgenden Tabelle 1 ist der prozentuale Abfall der Melaninwerte im Verlaufe von 14 Tagen aufgeführt.

**Tabelle 1**

| Verlauf der Melaninwerte (MX18 Mexameter®) in % | | | | | | |
|---|---|---|---|---|---|---|
| **Tage** | | | | | | |
| **P** | 0 | 4 | 7 | 9 | 11 | 14 |
| 1 | 100 | 95 | 88 | 84 | 81 | 62 |
| 2 | 100 | 92 | 90 | 86 | 79 | 60 |
| 3 | 100 | 90 | 86 | 80 | 74 | 64 |
| 4 | 100 | 89 | 83 | 77 | 72 | 58 |
| 5 | 100 | 93 | 87 | 81 | 76 | 62 |
| 6 | 100 | 96 | 89 | 83 | 72 | 59 |
| 7 | 100 | 90 | 85 | 78 | 68 | 58 |
| 8 | 100 | 93 | 89 | 85 | 72 | 60 |
| 9 | 100 | 91 | 88 | 84 | 74 | 59 |
| 10 | 100 | 94 | 87 | 80 | 71 | 61 |
| K 1 | 100 | 88 | 79 | 70 | 62 | 52 |
| K 2 | 100 | 85 | 70 | 63 | 58 | 48 |
| K 3 | 100 | 84 | 68 | 60 | 49 | 43 |

| | | | | | | |
|---|---|---|---|---|---|---|
| P = Proband-Nr. K = Kontrolle Nr. | | | | | | |

Aus der Tabelle ist klar ersichtlich, dass die Hautbräunung nach 7 Tagen noch immer deutlich über 80 % vom Ausgangswert lag, durchschnittlich bei etwa 87 %, während die Kontrolle (Auftragen einer Lotion bzw. Creme ohne die Phase C von Beispiel 2 bzw. die Phase D von Beispiel 1) bei etwa 72 % lag. Auch die weitere Verlängerung durch zusätzliche Komponenten gemäß Beispiel 2 zeigt Durchschnittswerte nach 11 Tagen von etwa 76 %, während die Kontrolle bei etwa 56 % lag. Daraus ist eine signifikante Bräunungsverlängerung ersichtlich.

## Patentansprüche

1. Verwendung eines kosmetischen Präparates enthaltend als wirksamen Bestandteil einen Komplex aus
0,1 bis 3,0 Gew-% Caffein,
0,2 bis 2 Gew-% Phototan® mit dem INCI-Namen Sorbitol (and) Arginin HCl (and) Ornithin HCl (and) Tyrosin (and) Silica und mit den Inhaltsstoffen: Sorbitol >50%, Arginine HCl 5 - 10%, Ornithine HCl 1 - 5%, Tyrosine 1 - 5% und Silica 0,1 - 1%, wobei diese %-Angaben auf das Gesamtgewicht Phototan® bezogen sind,
0,01 bis 1,0 Gew-% Kupfergluconat
und weitere kosmetische Hilfsstoffe, Trägerstoffe, weitere Wirkstoffe und Gemische davon bis zu 100 Gew-%, jeweils bezogen auf das Gesamtgewicht des Präparates, zur Verlängerung der Hautbräunung, die durch natürliche Sonneneinstrahlung entstanden ist.

2. Verwendung nach Anspruch 1,
**dadurch gekennzeichnet, dass** das kosmetische Präparat als weiteren Wirkstoff 0,01 bis 1,0 Gew-% der Aminosäure Glycin enthält.

3. Verwendung nach Anspruch 1,
**dadurch gekennzeichnet, dass** das kosmetische Präparat als weiteren Wirkstoff 0,001 bis 0,002 Gew-% eines Extraktes von Citrus aurantium-Früchten enthält, vorzugsweise 0,001 bis 0,0015 Gew-%.

4. Verwendung nach Anspruch 1,
**dadurch gekennzeichnet, dass** das kosmetische Präparat als weitere Wirkstoffe radikalfangende Komponenten enthält.

5. Verwendung nach Anspruch 1,
**dadurch gekennzeichnet, dass** das kosmetische Präparat als radikalfangende Komponente 0,4 bis 1,5 Gew-% eines Pflanzenextraktgemisches auf alkoholischer Basis enthält, bestehend aus den Extrakten von grünen Kaffeebohnen, Angelikawurzel, Camellia sinensis-Blättern und Pongamia pinnata.

6. Verwendung nach Anspruch 1, **dadurch gekennzeichnet, dass** das kosmetische Präparat vorzugsweise 0,1 bis 2,0, insbesondere 0,1 bis 0,5 Gew-% Caffein enthält.

7. Verwendung nach Anspruch 1, wobei das kosmetische Präparat wenigstens zweimal täglich mit einem Mindestabstand von 10 Stunden aufgetragen wird.

8. Verwendung nach Anspruch 7, **dadurch gekennzeichnet, dass** sie zusammen mit einem weiteren Wirkstoff erfolgt, wobei der Wirkstoff ausgewählt ist unter Glycin oder einem Extrakt von Citrus aurantium-Früchten.

## Claims

1. Use of a cosmetic preparation comprising as active ingredient a complex of
0.1 to 3.0 wt.-% caffeine,
0.2 to 2 wt.-% Phototan® with the INCI name Sorbitol (and) Arginine HCl (and) Ornithine HCl (and) Tyrosine (and) Silica and with the ingredients: sorbitol >50%, arginine HCl 5 - 10%, ornithine HCl 1 - 5%, tyrosine 1-5% and silica 0.1 - 1%, wherein these %-values are related to the overall weight of Phototan®,
0.01 to 1.0 wt.-% copper gluconate
and further cosmetic auxiliaries, excipients, further active substances and mixtures thereof to make 100 wt.-%, each relative to the overall weight of the preparation, for extension of tan which arised from natural sun radiation.

2. Use according to claim 1, **characterized in that** the cosmetic preparation comprises 0.01 to 1.0 wt.-% of the amino acid glycine as further active substance.

3. Use according to claim 1, **characterized in that** the cosmetic preparation comprises as further active substance 0.001 to 0.002 wt.-% of an extract of Citrus aurantium fruits, preferably 0.001 to 0.0015 wt.-%.

4. Use according to claim 1, **characterized in that** the cosmetic preparation comprises radical-scavenging components as further active substances.

5. Use according to claim 1, **characterized in that** the cosmetic preparation comprises as radical-scavenging component 0.4 to 1.5 wt.-% of an alcohol-based plant extract mixture consisting of extracts of green coffee beans, angelica roots, Camellia sinensis leaves and Pongamia pinnata.

6. Use according to claim 1, **characterized in that** the cosmetic preparation comprises preferably 0.1 to 2.0, particularly 0.1 to 0.5 wt.-% caffeine.

7. Use according to claim 1, wherin the cosmetic preparation is applied at least twice a day with a minimum interval of 10 hours.

8. Use according to claim 7, **characterized in that** it is applied together with an additional active substance, which active substance is selected among glycine or an extract of Citrus aurantium fruits.

## Revendications

1. Utilisation d'une préparation cosmétique contenant, en tant que composant actif, un complexe constitué de
0,1 à 3,0 % en poids de caféine,
0,2 à 2 % en poids de Phototan® ayant la désignation INCI sorbitol (and) arginine HCl (and) ornithine HCl (and) tyrosine (and) silica et les ingrédients suivants : sorbitol > 50 %, arginine HCl 5 à 10 %, ornithine HCl 1 à 5 %, tyrosine 1 à 5 % et silice 0,1 à 1 %, les pourcentages étant exprimés par rapport au poids total du Phototan®.
0,01 à 1,0 % en poids de gluconate de cuivre
et d'autres agents auxiliaires cosmétiques, excipients, d'autres principes actifs et leurs mélanges jusqu'à atteindre 100 % en poids, pour chacun d'entre eux exprimé par apport au poids total de la préparation, pour prolonger le bronzage de peau résultant d'une exposition au rayonnement naturel du soleil.

2. Utilisation selon la revendication 1,
**caractérisée en ce que** ladite préparation cosmétique contient, en tant qu'autre principe actif, 0,01 à 1,0 % en poids de l'acide aminé glycine.

3. Utilisation selon la revendication 1,
**caractérisée en ce que** ladite préparation cosmétique contient, en tant qu'autre principe actif, 0,001 à 0,002 % en poids d'un extrait des fruits de Citrus aurantium, préférentiellement 0,001 à 0,0015 % en poids.

4. Utilisation selon la revendication 1,
**caractérisée en ce que** ladite préparation cosmétique contient, en tant qu'autres principes actifs, des constituants capables de neutraliser des radicaux.

5. Utilisation selon la revendication 1,
**caractérisée en ce que** ladite préparation cosmétique contient, en tant que constituant capable de neutraliser des radicaux, 0,4 à 1,5 % en poids d'un mélange d'extraits végétaux à base d'alcool, constitué d'extraits de grains de café verts, de la racine d'Angelica, de feuilles de Camellia sinensis ainsi que de Pongamia pinnata.

6. Utilisation selon la revendication 1, **caractérisée en ce que** ladite préparation cosmétique contient préférentiellement 0,1 à 2,0, notamment 0,1 à 0,5 % en poids, de caféine.

7. Utilisation selon la revendication 1, ladite préparation cosmétique étant appliquée au moins deux fois par jour, avec écart d'au moins 10 heures.

8. Utilisation selon la revendication 7, **caractérisée en ce qu'**elle comporte l'association avec un autre principe actif, ledit principe actif étant choisi parmi la glycine ou un extrait des fruits de Citrus aurantium.
